# EUROPEAN PATENT APPLICATION

(11) **EP 4 230 727 A1**
(43) Date of publication of application: **23.08.2023**
(21) Application number: 21895083.0
(22) Date of filing: 17.11.2021
(51) Int. Cl.: C12N 5/071, C12N 5/00, C12M 3/00

(54) **DECELLULARIZED KIDNEY EXTRACELLULAR MATRIX-DERIVED SCAFFOLD FOR CULTURING AND TRANSPLANTING KIDNEY ORGANOID, AND PREPARATION METHOD THEREFOR**

(30) Priority: 17.11.2020 KR 20200153719; 16.11.2021 KR 20210157357
(71) Applicant: Cellartgen Inc., Seoul 03722 (KR)
(72) Inventor: CHO, Seung Woo, Seoul 03722 (KR); KIM, Yu Heun, Seoul 03722 (KR); CHOI, Yi Sun, Seoul 03722 (KR)
(74) Representative: Manitz Finsterwald Patent- und Rechtsanwaltspartnerschaft mbB
(86) International application number: PCT/KR2021/016827
(87) International publication number: WO 2022/108315

(57) **Abstract**

The present disclosure relates to a scaffold for culture and transplantation of a kidney organoid using a decellularized kidney tissue (KEM).

## Description

### TECHNICAL FIELD

The present disclosure relates to a kidney extracellular matrix-derived scaffold for culture and transplantation of a kidney organoid and a method of preparing the same.

### BACKGROUND

A conventional two-dimensional (2D) cell culture method is limited in implementing an in vivo microenvironment and thus has a low culture efficiency. Therefore, a 2D cell line is a limited in vitro model. A three-dimensional organoid culture technology has recently attracted a lot of interest as a new alternative to overcome these limitations. The organoid is the technology that has been rapidly growing worldwide and can be applied as a tissue analogue to various application fields, such as drug screening, drug toxicity evaluation, disease modeling, cell therapeutic agent and tissue engineering. The organoid is a three-dimensional structure composed of various cells of a specific human organ and tissue and can implement complicated interactions between them. Therefore, it can be applied as a more accurate in vitro model platform than conventionally used drug evaluation models such as 2D cell line models or animal models.

Platforms for organoids derived from various organs have been established worldwide and relevant research is still being actively conducted. Currently, Matrigel is used as a culture scaffold to culture various types of organoids. However, since Matrigel is an extracellular matrix component extracted from mouse sarcoma tissue, it is difficult to maintain the quality of products uniformly and it is expensive and has a safety problem such as metastasis of animal pathogens and viruses. Therefore, Matrigel as an organoid culture system has a lot of problems to be solved. In particular, as a material derived from cancer tissue, it cannot provide an optimal tissue-specific microenvironment necessary for culturing a specific tissue organoid. There have been some studies on the development of polymer-based hydrogels to replace Matrigel, but no material has been reported that can replace Matrigel.

A kidney organoid is produced by extracting adult stem cells from kidney tissue and culturing them or produced from pluripotent stem cells, such as human induced pluripotent stem cells or embryonic stem cells. Since Matrigel used for kidney organoid culture cannot implement a complex kidney tissue-specific microenvironment in vivo, kidney organoid differentiation efficiency and function need to be improved. Accordingly, there is a desperate need for the development of a new culture system for producing a more matured and functional kidney organoid.

There is no suitable treatment for intractable kidney diseases such as end-stage renal failure and chronic nephritis, so patients require hemodialysis for the rest of their life or require a kidney transplant. Therefore, the intractable kidney diseases are serious diseases that inhibit everyday activities, degrade the quality of life of the patients, and cause great suffering due to intake of immunosuppressants and side effects after the kidney transplant. Therefore, it is very important to develop a system, which can efficiently culture kidney organoids as a precise in vitro model for development of therapeutic agents for kidney disease, from the point of view of health care.

In the present disclosure, a hydrogel matrix composed of kidney tissue-specific extracellular matrix components was prepared through a decellularization process of kidney tissue and applied to kidney organoid culture. In the present disclosure, the decellularization process is simplified compared with the conventional decellularization method. Thus, a matrix can be very easily produced, and the kidney tissue-specific extracellular matrix components and growth factors can be well preserved. Therefore, it is confirmed that the hydrogel matrix can induce efficient growth and differentiation of kidney organoids, which verifies that the hydrogel matrix can replace the conventional Matrigel as a culture matrix.

### DISCLOSURE OF THE INVENTION

### PROBLEMS TO BE SOLVED BY THE INVENTION

The present disclosure is conceived to obtain a large amount of decellularized tissue through a chemical treatment of a porcine kidney tissue, prepare a hydrogel scaffold based on the decellularized tissue and apply it to kidney organoid culture.

However, the problems to be solved by the present disclosure are not limited to the above-described problems. Although not described herein, other problems to be solved by the present disclosure can be clearly understood by a person with ordinary skill in the art from the following descriptions.

### MEANS FOR SOLVING THE PROBLEMS

An aspect of the present disclosure provides a scaffold for culture and transplantation of a kidney organoid using a kidney extracellular matrix (KEM).

In an embodiment of the present disclosure, the KEM may be prepared by using a mixed solution of Triton X-100 and ammonium hydroxide.

In an embodiment of the present disclosure, a concentration of the KEM in the scaffold may be from 1 mg/ml to 10 mg/ml.

Another aspect of the present disclosure provides a method of preparing a scaffold for culture and transplantation of a kidney organoid, including: a process (1) of crushing an isolated kidney tissue; and a process (2) of treating the crushed kidney tissue with Triton X-100 and ammonium hydroxide for decellularization to prepare a decellularized KEM.

In an embodiment of the present disclosure, the method may further include, after the process (2), a process (3) of lyophilizing the decellularized KEM to prepare a lyophilized KEM.

In an embodiment of the present disclosure, the method may further include, after the process (3), a process (4) of forming a scaffold for culture and transplantation of a kidney organoid in the form of a hydrogel with the lyophilized KEM.

In an embodiment of the present disclosure, in the process (4), the lyophilized KEM may be dissolved in a pepsin solution and a pH of the solution may be adjusted to form the hydrogel.

Yet another aspect of the present disclosure provides a method of culturing a kidney organoid in the above-described scaffold or in a scaffold prepared by the above-described preparation method.

### EFFECTS OF THE INVENTION

Since the decellularized scaffold developed in the present disclosure enables efficient culture of kidney organoids, it is expected to be widely used in the medical industry, such as new drug development, drug toxicity and efficacy evaluation, and patient-specific drug selection, in substitution for the conventional Matrigel, which has various problems. Accordingly, it is expected to improve the quality of life of people in terms of health care and create high added value in industrial and economic terms.

The decellularized kidney tissue-derived artificial matrix scaffold is expected to be widely used in various fields, such as disease modeling research that implements various intractable kidney diseases (acute and chronic nephritis, renal failure, etc.) in vitro and investigate the mechanisms thereof, and establishment of a transplantation treatment platform. Since the prevalence of such intractable kidney diseases has increased significantly in recent years, a lot of research is needed. Therefore, the decellularized kidney tissue-derived artificial matrix scaffold can create profits as a research material for related basic research.

The kidney organoid has an endless potential as a disease model as well as a cell therapeutic agent and a tissue regenerative agent for regenerative medicine. End-stage renal failure patients cannot enjoy their ordinary life due to hemodialysis. However, if fundamental treatment of kidney diseases is possible through kidney organoid transplantation treatment using the decellularized kidney-derived scaffold developed in the present disclosure, the quality of life of the patients will be greatly improved and relevant costs can be significantly reduced.

The artificial scaffold developed in the present disclosure can be applied to culture of stem cell-derived kidney organoids and renal cancer organoids and thus can contribute to the construction of a disease model customized for intractable disease and cancer patient and can also be used as a precision medicine platform technology. Further, it is expected to create enormous added value in consideration of the size of the precision medicine market that has been rapidly increasing in recent years.

Overall, as described above, for culture and application of a kidney organoid, Matrigel is essentially required. Compared with Matrigel, the artificial scaffold developed in the present disclosure is verified to have advantages in terms of safety and cost, showing better functionality as a culture system than Matrigel. Therefore, it is expected to replace Matrigel and create huge economic profits.

### BRIEF DESCRIPTION OF THE DRAWINGS

**FIG. 1a** shows a process of fabricating a decellularized kidney tissue-derived scaffold (kidney extracellular matrix: KEM) for kidney organoid culture.
**FIG. 1b** shows a process of fabricating a decellularized kidney tissue-derived scaffold (kidney extracellular matrix: KEM) for kidney organoid culture.
**FIG. 2a** shows the result of analyzing the KEM for kidney organoid culture.
**FIG. 2b** shows the result of analyzing the KEM for kidney organoid culture.
**FIG. 2c** shows the result of analyzing the KEM for kidney organoid culture.
**FIG. 2d** shows the result of analyzing the KEM for kidney organoid culture.
**FIG. 3** shows an analysis of properties depending on the concentration of the KEM in a KEM hydrogel scaffold.
**FIG. 4** shows a proteomics analysis of the KEM for kidney organoid culture.
**FIG. 5a** shows the result of comparative analysis of proteomes of the KEM for kidney organoid culture and Matrigel.
**FIG. 5b** shows the result of comparative analysis of proteomes of the KEM for kidney organoid culture and Matrigel.
**FIG. 5c** shows the result of comparative analysis of proteomes of the KEM for kidney organoid culture and Matrigel.
**FIG. 6a** shows the analysis results for selecting an optimal concentration of the KEM hydrogel scaffold for kidney organoid culture.
**FIG. 6b** shows the analysis results for selecting an optimal concentration of the KEM hydrogel scaffold for kidney organoid culture.
**FIG. 6c** shows the analysis results for selecting an optimal concentration of the KEM hydrogel scaffold for kidney organoid culture.
**FIG. 7** shows the results of analyzing the expression of proliferation and differentiation markers of a kidney organoid cultured in the KEM hydrogel scaffold (cell immunostaining analysis).
**FIG. 8** shows a growth pattern of the kidney organoid cultured in the KEM hydrogel scaffold.
**FIG. 9a** shows the results of analyzing a kidney organoid cultured for a long time in the KEM.
**FIG. 9b** shows the results of analyzing a kidney organoid cultured for a long time in the KEM.
**FIG. 9c** shows the results of analyzing a kidney organoid cultured for a long time in the KEM.
**FIG. 10a** shows the results of verifying the possibility of long-term storage of the KEM.
**FIG. 10b** shows the results of verifying the possibility of long-term storage of the KEM.
**FIG. 10c** shows the results of verifying the possibility of long-term storage of the KEM.
**FIG. 10d** shows the results of verifying the possibility of long-term storage of the KEM.
**FIG. 11a** shows the results of verifying the possibility of long-term storage of the KEM.
**FIG. 11b** shows the results of verifying the possibility of long-term storage of the KEM.
**FIG. 11c** shows the results of verifying the possibility of long-term storage of the KEM.
**FIG. 11d** shows the results of verifying the possibility of long-term storage of the KEM.
**FIG. 12a** shows the results of verifying the tissue-specific effect of the KEM for kidney organoid culture.
**FIG. 12b** shows the results of verifying the tissue-specific effect of the KEM for kidney organoid culture.
**FIG. 12c** shows the results of verifying the tissue-specific effect of the KEM for kidney organoid culture.
**FIG. 13a** shows the results of analyzing the functionality of the kidney organoid cultured in the KEM.
**FIG. 13b** shows the results of analyzing the functionality of the kidney organoid cultured in the KEM.
**FIG. 14a** shows the results of constructing a kidney fibrosis model using the kidney organoid cultured in the KEM.
**FIG. 14b** shows the results of constructing a kidney fibrosis model using the kidney organoid cultured in the KEM.
**FIG. 14c** shows the results of constructing a kidney fibrosis model using the kidney organoid cultured in the KEM.
**FIG. 15** shows the result of verifying biocompatibility of the KEM.
**FIG. 16** shows the results of in vivo transplantation of the kidney organoid using the KEM.
**FIG. 17a** shows the results of in vivo transplantation of the kidney organoid using the KEM.
**FIG. 17b** shows the results of in vivo transplantation of the kidney organoid using the KEM.

### BEST MODE FOR CARRYING OUT THE INVENTION

Hereafter, the present disclosure will be described in detail with reference to the accompanying drawings. However, it is to be noted that the present disclosure is not limited to examples described herein but can be embodied in various other ways. It is to be understood that the term "comprises or includes" and/or "comprising or including" used in the document means that one or more other components, steps, operation and/or existence or addition of elements are not excluded in addition to the described components, steps, operation and/or elements unless context dictates otherwise.

Unless otherwise indicated, the practice of the disclosure involves conventional techniques commonly used in molecular biology, microbiology, protein purification, protein engineering, protein and DNA sequencing, and recombinant DNA fields, which are within the skill of the art. Such techniques are known to a person with ordinary skill in the art and are described in numerous standard texts and reference works.

Unless otherwise defined herein, all technical and scientific terms used herein have the same meaning as commonly understood by a person with ordinary skill in the art to which this disclosure belongs.

Various scientific dictionaries that include the terms included herein are well known and available to those in the art. Although any methods and materials similar or equivalent to those described herein find use in the practice or testing of the disclosure, some preferred methods and materials are described. It is to be understood that this disclosure is not limited to the particular methodology, protocols, and reagents described, as these may vary, depending upon the context in which they are used by a person with ordinary skill in the art. Hereinafter, the present disclosure will be described in more detail.

An aspect of the present disclosure provides a scaffold for culture and transplantation of a kidney organoid using a kidney extracellular matrix (KEM).

The term "extracellular matrix" refers to a natural scaffold for cell growth that is prepared by decellularization of tissue found in mammals and multicellular organisms. The extracellular matrix can be further processed through dialysis or crosslinking.

The extracellular matrix may be a mixture of structural or non-structural biomolecules including, but not limited to, collagens, elastins, laminins, glycosaminoglycans, proteoglycans, antimicrobials, chemoattractants, cytokines and growth factors.

In mammals, the extracellular matrix may contain about 90% collagen in various forms. The extracellular matrices derived from various living tissues may differ in their overall structure and composition due to the unique role needed for each tissue.

The term "derive" or "derived" refers to a component obtained from any stated source by any useful method.

In an embodiment of the present disclosure, the KEM may be prepared by using a mixed solution of Triton X-100 and ammonium hydroxide.

In an embodiment of the present disclosure, a concentration of the KEM in the scaffold may be from 1 mg/ml to 10 mg/ml, specifically from 1 mg/ml to 7 mg/ml. The concentration of the KEM may be, for example, from 1 mg/ml to 7 mg/ml, from 1 mg/ml to 5 mg/ml, from 1 mg/ml to 3 mg/ml, from 3 mg/ml to 7 mg/ml, from 3 mg/ml to 5 mg/ml, or from 5 mg/ml to 7 mg/ml. In an example, the concentration of the KEM may be 1 mg/ml, 3 mg/ml, 5 mg/ml, or 7 mg/ml. The KEM contained with a concentration out of the above-described range may make it impossible to achieve the intended effect of the present disclosure.

The scaffold includes a three-dimensional hydrogel prepared based on the KEM obtained by decellularization, and can be effectively used for kidney organoid culture.

The decellularized kidney tissue contains actual tissue-specific extracellular matrix components and thus can provide the physical, mechanical and biochemical environment of the tissue, and is highly efficient in enhancing differentiation into kidney tissue cells and tissue-specific functionality.

The term "organoid" refers to an ultraminiature body organ prepared in the form of an artificial organ by culturing cells derived from tissues or pluripotent stem cells in a 3D form.

The organoid is a three-dimensional tissue analog that contains organ-specific cells which originate from stem cells and self-organize (or self-pattern) in a similar manner to the in vivo condition. The organoid can be developed into a specific tissue by patterning a restricted element (for example, a growth factor).

The organoid can have the intrinsic physiological properties of the cells and can have an anatomical structure that mimics the original state of a cell mixture (including all remaining stem cells and the neighboring physiological niche as well as limited cell types). A three-dimensional culture method allows the organoid to be better arranged in terms of cell to cell functions and to have an organ-like form with functionality and a tissue-specific function.

Another aspect of the present disclosure provides a method of preparing a scaffold for culture and transplantation of a kidney organoid, including: a process (1) of crushing an isolated kidney tissue; and a process (2) of treating the crushed kidney tissue with Triton X-100 and ammonium hydroxide for decellularization to prepare a decellularized KEM.

The process (1) is a process of crushing an isolated kidney tissue, and the kidney tissue may be isolated from a known animal. Examples of the animal may include cattle, pigs, monkeys, humans, etc. Also, in the present disclosure, the isolated kidney tissue is crushed and then decellularized, which results in high efficiency in decellularization. The isolated kidney tissue may be crushed by a known method. According to the present disclosure, the kidney tissue was crushed and decellularized, and, thus, the cells can be removed more efficiently at a higher level.

The process (2) is a process of treating the crushed kidney tissue with Triton X-100 and ammonium hydroxide for decellularization to prepare a decellularized KEM. According to the method of the present disclosure unlike a conventional decellularization method, the tissue is treated with Triton X-100 and ammonium hydroxide only to minimize damage to the tissue, and, thus, it is possible to preserve various proteins more in the kidney tissue. For example, the decellularization may be performed by stirring the crushed kidney tissue with Triton X-100 and ammonium hydroxide.

In an embodiment of the present disclosure, the method may further include, after the process (2), a process (3) of lyophilizing the decellularized KEM to prepare a lyophilized KEM.

The process (3) is a process of lyophilizing the decellularized KEM to prepare a lyophilized KEM. After drying, the lyophilized KEM may be exposed to electron beam, gamma radiation, ethylene oxide gas, or supercritical carbon dioxide for sterilization.

In an embodiment of the present disclosure, the method may further include, after the process (3), a process (4) of forming a scaffold for culture and transplantation of a kidney organoid in the form of a hydrogel with the lyophilized KEM.

The process (4) is a process of forming a scaffold for culture and transplantation of a kidney organoid in the form of a hydrogel with the lyophilized KEM. The process (4) may be performed through gelation. Specifically, the lyophilized KEM may be dissolved in a pepsin solution and a pH of the solution may be adjusted to form a hydrogel. The decellularized KEM may be crosslinked to prepare a three-dimensional hydrogel-type scaffold, and the gelated scaffold can be used in various ways in the fields related to tests, screening and organoid culture.

The term "hydrogel" is a material in which a liquid that contains water as a dispersion medium is hardened through a sol-gel phase transition to lose fluidity and to form a porous structure. The hydrogel can be formed by causing a hydrophilic polymer that has a three-dimensional network structure and a microcrystalline structure to contain water and to be expanded.

The gelation may be performed at a temperature of 37°C for 30 minutes after dissolving the lyophilized KEM in an acidic solution with a protease such as pepsin or trypsin and adjusting a pH, specifically setting a neutral pH in an electrolyte state of 1X PBS buffer by using 10X PBS and 1 M NaOH.

Yet another aspect of the present disclosure provides a method of culturing a kidney organoid in the above-described scaffold or a scaffold prepared by the above-described preparation method.

The conventional Matrigel-based culture system is an extract derived from animal cancer tissue, has a large difference between the batches, cannot mimic the actual kidney environment, and exhibits insufficient efficiency in differentiation or development into a kidney organoid. However, the scaffold can create a kidney tissue-like environment and thus is suitable for kidney organoid culture.

The culture refers to a process of maintaining and growing cells under suitable conditions, and the suitable conditions may refer to, for example, the temperature, nutrient availability, atmospheric CO₂ level and cell density at which the cells are maintained.

Appropriate culture conditions for maintaining, proliferating, expanding and differentiating different types of cells are known in the art and are documented. Suitable conditions for formation of the organoid may facilitate or allow cell differentiation and formation of a multicellular structure.

### MODE FOR CARRYING OUT THE INVENTION

Since the decellularized kidney tissue-derived extracellular matrix scaffold developed in the present disclosure can be fabricated through minimized chemical treatment, there is less damage to tissue-specific components than in a scaffold prepared by the conventional decellularization method. Also, the decellularized kidney tissue-derived extracellular matrix scaffold is more efficient in terms of production time and cost reduction, and has an advantage of easy mass production. Therefore, it is expected to be more advantageous in terms of commercialization than conventional decellularized matrixes.

It was confirmed that in the decellularized kidney tissue-derived scaffold developed in the present disclosure, all immunogenic cells were removed, various extracellular matrix components and growth factors contained in the actual kidney tissue were well preserved. Through proteomics, important extracellular matrix components and related proteins important in the kidney tissue were identified. Therefore, the decellularized kidney tissue-derived matrix provided a kidney tissue-specific microenvironment and enabled efficient culture of kidney organoids.

According to the present disclosure, it was confirmed that formation and differentiation of a kidney organoid was successfully induced in the developed decellularized kidney tissue-derived hydrogel. Decellularized scaffolds of various extracellular matrix concentrations were prepared and applied to kidney organoid culture to select an optimal concentration for the most efficient kidney organoid culture.

When a kidney organoid cultured in the developed decellularized scaffold and a kidney organoid cultured in a Matrigel scaffold used as a control were compared and analyzed, it was confirmed that differentiation of the kidney organoid cultured in the decellularized scaffold was further enhanced. Based on this result, it was confirmed that kidney organoid cultured in the decellularized scaffold can reproduce the actual kidney tissue more accurately and precisely than the kidney organoid cultured by the conventional method. As a result, it was confirmed that the decellularized kidney tissue-derived scaffold actually contributes to efficient differentiation and development of kidney organoids, which demonstrates its potential as an alternative to Matrigel.

Hereinafter, Examples will be presented for understanding of the present disclosure. However, the following Examples are illustrative only for better understanding of the present disclosure but do not limit the present disclosure.

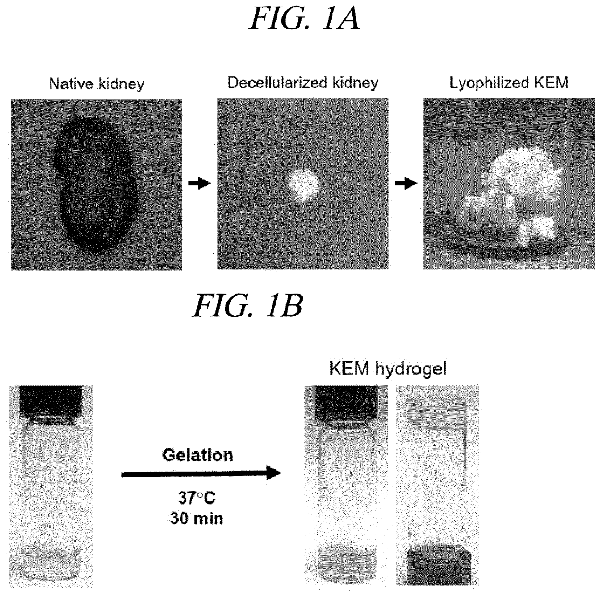

### Fabrication of kidney extracellular matrix (KEM) for kidney organoid culture (FIG. 1)

A kidney extracellular matrix (KEM) was fabricated from a porcine kidney tissue to prepare a culture scaffold for kidney organoid culture. The present decellularization process was different from conventional decellularization methods in that it was performed using only a mixed solution of 1% Triton X-100 and 0.1% ammonium hydroxide, which are more relaxed conditions than those in the conventional methods. Therefore, it is possible to minimize damage to the tissue, and, thus, it is possible to preserve various extracellular matrix and growth factor proteins more in the kidney tissue. Also, according to the present disclosure, the kidney tissue was finely cut and then decellularized, and, thus, the cellular components (DNA) were more effectively and completely removed.

(A) After the porcine kidney tissue was finely cut, most of the cells in the tissue were removed through a series of chemical treatments for decellularization. Then, the decellularized tissue was lyophilized to obtain a lyophilized KEM in the form of powder.

(B) 10 mg of the lyophilized KEM powder containing the extracellular matrix of the kidney tissue was treated with a 4 mg/ml pepsin solution (a solution of 4 mg of porcine gastric mucosa-derived pepsin powder dissolved in 1 ml of 0.02 M HCl), followed by solutionization at room temperature for 48 hours. After 10X PBS and 1M NaOH were added at an appropriate ratio to the solutionized KEM and evenly mixed to set a neutral pH in an electrolyte state of 1X PBS buffer. Finally, gelation was induced at 37°C for 30 minutes in an incubator. A KEM hydrogel formed by inducing gelation in a glass container did not flow down even when the container was overturned, which confirmed that a solidified hydrogel was prepared through temperature-dependent crosslinking.

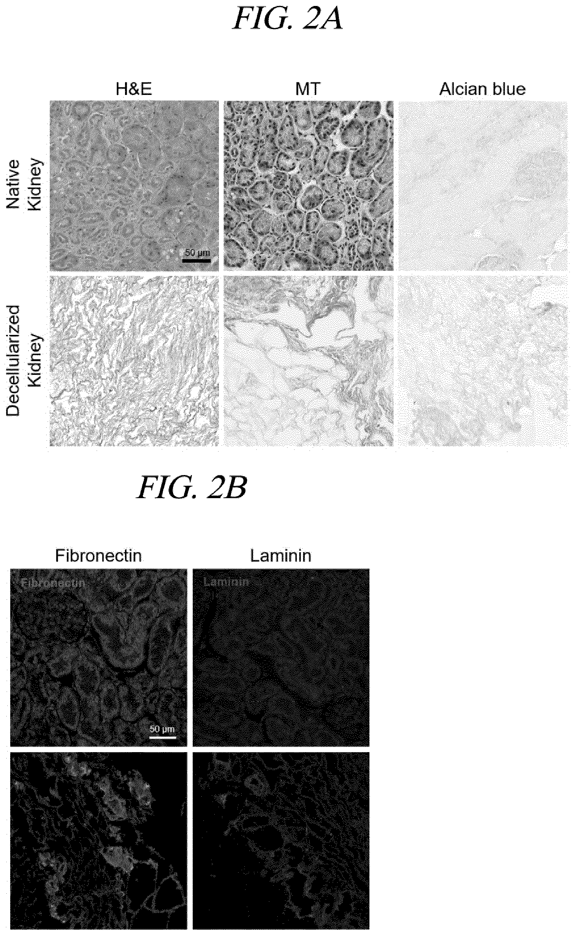

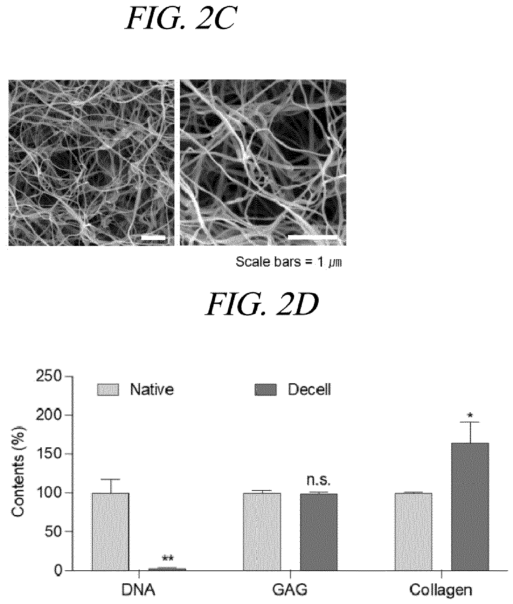

### Analysis of KEM scaffold for kidney organoid culture (FIG. 2)

A KEM was fabricated from a porcine kidney tissue and the properties thereof were analyzed.

(A) H&E staining was performed to confirm that most of the cell nucleuses in the tissue were removed, but the overall structure was well preserved after the decellularization process. Also, Masson's trichrome staining was performed to confirm that collagen was well preserved. Further, Alcian blue staining was performed to confirm that the glycosaminoglycans (GAGs) were well preserved.

(B) Immunostaining was performed to confirm whether ECM proteins are well preserved in the kidney tissue even after the decellularization process. As a result, it was confirmed that the ECM proteins such as fibronectin, laminin, etc. were well preserved even after the decellularization process.

(C) Through scanning electron microscopy (SEM), it was confirmed that the KEM hydrogel had a collagen nanofiber-specific porous microstructure. Therefore, it was confirmed that the KEM hydrogel can provide a three-dimensional microenvironment suitable for kidney organoid culture.

(D) Quantitative analysis of DNAs and glycosaminoglycans (GAGs) was performed to the kidney tissue before decellularization (Native) and after decellularization (Decell). As a result, 96.8% or more of the DNAs were removed after decellularization, which confirmed that the cells were efficiently removed. Also, it was confirmed that the GAGs were well preserved at a similar level to that in the actual kidney tissue, and the collagen was well preserved.

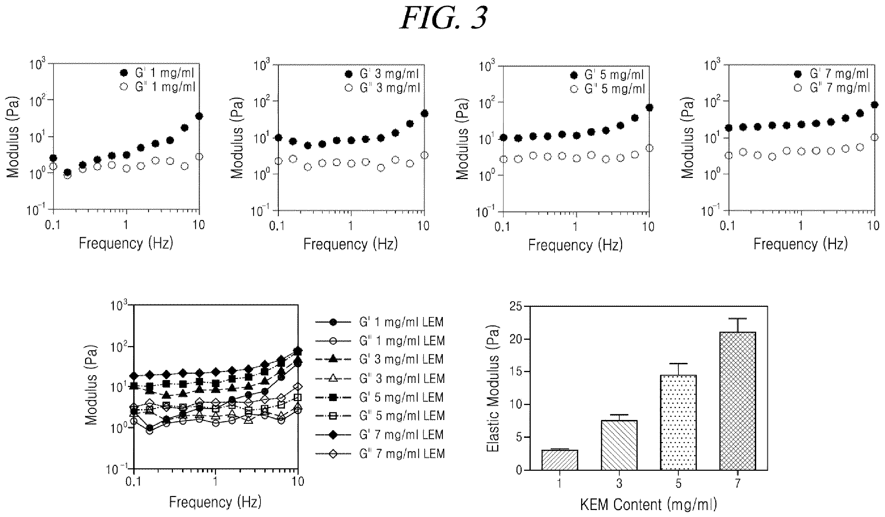

### Analysis of properties depending on concentration of KEM in KEM hydrogel scaffold (FIG. 3)

The KEM was used to fabricate hydrogel scaffolds with four different concentrations, and changes in properties depending on the concentration were measured. All the gels with different concentrations were fabricated by crosslinking for 30 minutes in a 37°C incubator. It was confirmed that a storage modulus G' value was consistently higher than a loss modulus G" value at all concentrations including the lowest concentration of 1 mg/ml, and, thus, a stable polymer network was formed through crosslinking in the hydrogel. It was confirmed that as the KEM concentration increased, the mechanical properties (modulus) also increased.

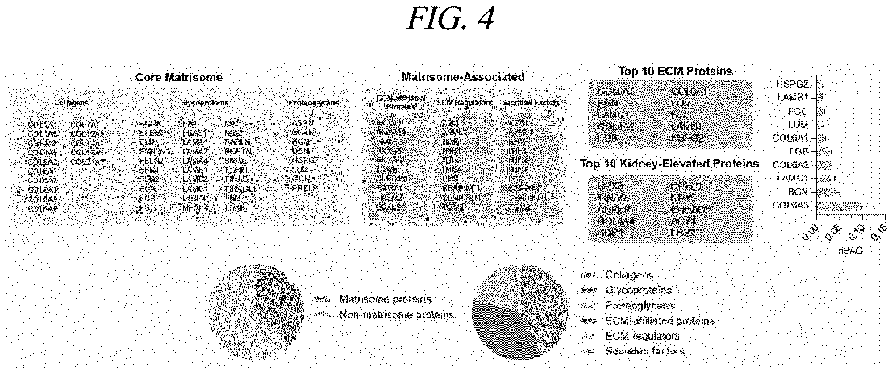

### Proteomics of KEM for kidney organoid culture (FIG. 4)

Proteomics was performed to identify the extracellular matrix components contained in the KEM scaffold.

The proteomics confirmed that the fabricated KEM hydrogel contains various extracellular matrix components, such as collagens, glycoproteins, proteoglycans, etc., as well as various secreted factors, such as growth factor, coagulation factors, cytokines, etc., which are secreted from cells in the tissue and bound to mainly the extracellular matrixes.

Collagen type VI (COL6), which accounts for a large portion among the top 10 ECMs constituting the KEM hydrogel, is a main component of the renal glomerular extracellular matrix and plays an important role in the formation of kidney tissue, and laminin (LAM) glycoprotein as a main component of the basement membrane is involved in kidney cell and tissue differentiation, homeostasis and survival. Also, biglycan (BGN) proteoglycans play an important role as a mediator in the regulation of renal immune responses.

Also, it was conformed that the KEM scaffold contains proteins (Top 10 kidney-elevated proteins) which are expressed more than 4-fold in the kidney tissue compared with other tissues.

Therefore, it is expected that these various proteins present in the actual kidney tissue and observed in the KEM hydrogel can induce the formation and development of a kidney organoid.

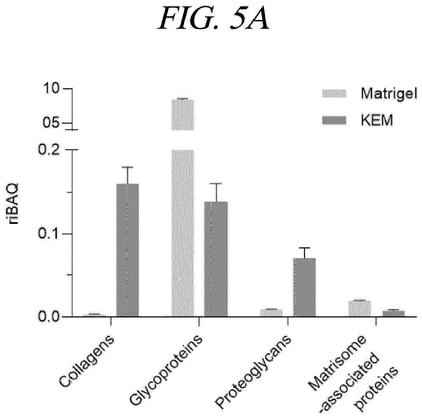

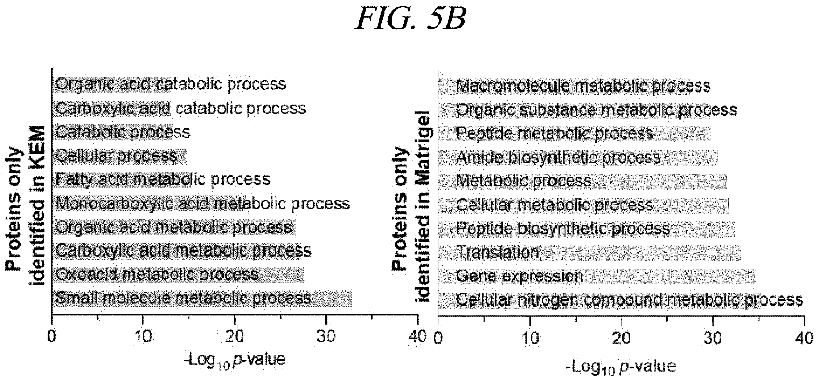

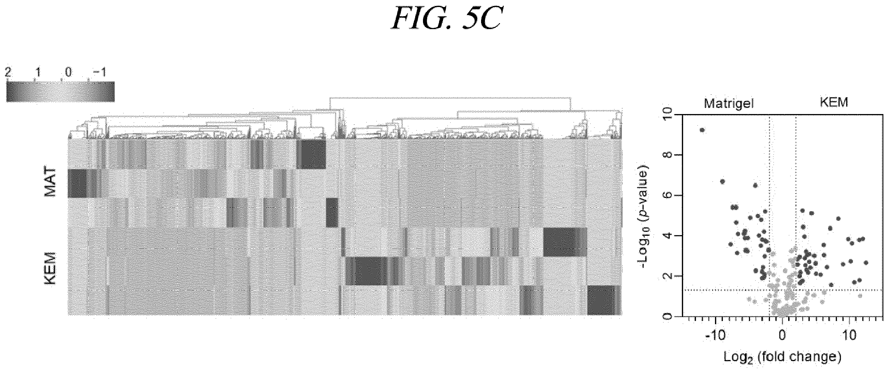

### Comparative analysis of proteome of KEM for kidney organoid culture and Matrigel scaffold (FIG. 5)

A comparative analysis was performed to proteomes of the KEM for kidney organoid culture and proteomes of the Matrigel scaffold.

(A) As a result of comparing the components of the KEM scaffold and Matrigel, it was confirmed that Matrigel is mostly composed of glycoproteins, whereas the KEM is composed of various collagens including glycoproteins, and proteoglycans.

(B) The functions of the top 10 ECM components included in the KEM scaffold were compared with those of the conventional Matrigel scaffold through gene ontology. As a result, it was confirmed that a greater amount of proteins related to nitrogen compound metabolism involved by the kidney is present in the KEM scaffold than in Matrigel.

(C) As a result of comparing the protein expression pattern between Matrigel and the KEM through a heatmap and a volcano plot, it was confirmed that the compositions of the proteins included in the two scaffolds are very different from each other. Also, through the volcano plot analysis showed a remarkable difference in expression tendency of proteins significantly expressed in a greater amount in each scaffold.

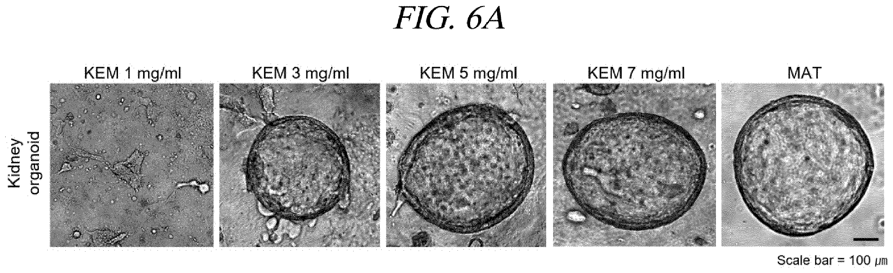

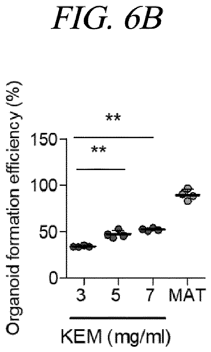

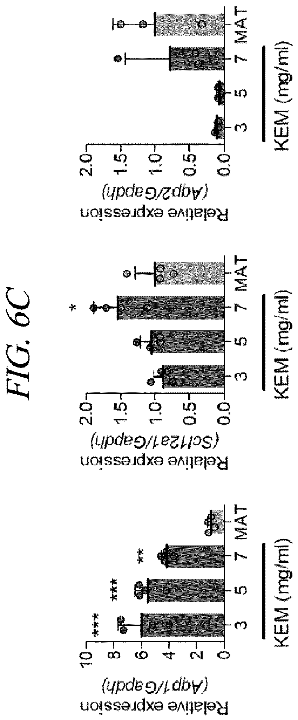

### Selection of optimal concentration of KEM hydrogel scaffold for kidney organoid culture (FIG. 6)

Tubular fragments were extracted from a mouse kidney tissue. In order to select the KEM hydrogel with the most suitable concentration for kidney organoid culture, KEM hydrogels with various concentration conditions were prepared. The extracted tubular fragments were evenly mixed with each of the KEM hydrogel scaffolds with the respective concentrations, followed by three-dimensional culture to induce the formation of kidney organoids. Subculture was performed on day 7 of cultivation, followed by further culture for 5 days. The shape, formation efficiency, and gene expression of the kidney organoids formed at the respective KEM concentrations were checked on day 12 of cultivation. Matrigel was used as a control group.

(A) Kidney organoids were formed in the KEM hydrogels with the concentrations except the concentration of 1 mg/ml, and it was confirmed that they were formed in a spherical shape similar to that in the organoid cultured in Matrigel used as a control group. No organoid was formed in the hydrogel with a concentration of 1 mg/ml.

(B) The formation efficiency was measured by measuring the number of organoids on each of day 0 and day 5 of subculture and expressed as a ratio. As a result of comparing the kidney organoids for the respective KEM concentrations in terms of formation efficiency, it was confirmed that the KEM hydrogels exhibited generally lower formation efficiency than Matrigel, but the KEM hydrogel with a concentration of 7 mg/ml exhibited the highest formation efficiency.

(C) After the kidney organoids were cultured for 12 days in the KEM hydrogels with the respective concentrations, the gene expression levels of differentiation markers were compared by quantitative qPCR analysis. Aqp1 (proximal tubule cell), a gene expressed in specific cells of the kidney, tends to decrease as the concentration of KEM increases, but is significantly higher than that of the Matrigel group at all concentrations of KEM. Also, the expression level of Scl12a1 (loop of Henle cell) was generally similar to that of the Matrigel group, but it was significantly increased in the KEM with a concentration of 7 mg/ml. Further, the Aqp2 (collecting duct cell) gene was expressed in the KEM with a concentration of 7 mg/ml at a level most similar to that in the Matrigel group.

Through the above analyses, it was confirmed that organoids can be cultured at all concentrations except the KEM hydrogel with a concentration of 1 mg/ml. Overall, it was confirmed that the kidney organoid exhibited the highest differentiation potency at a KEM concentration of 7 mg/ml.

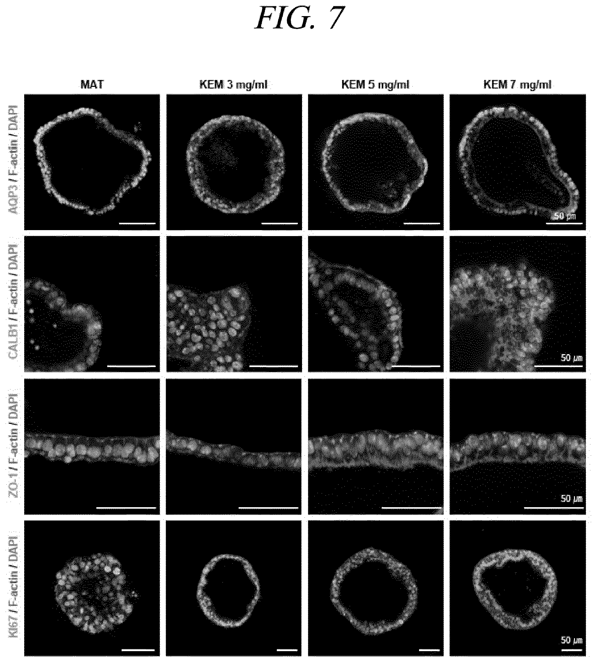

### Analysis of expression of proliferation and differentiation markers of kidney organoid cultured in KEM hydrogel scaffold (cell immunostaining analysis) (FIG. 7)

On day 12 of culture, immunostaining was performed to analyze the expression of various markers. As a result, it was confirmed that both AQP3 (collecting duct cell) and CALB1 (distal tubule cell), kidney organoid-specific differentiation markers, were well expressed at similar levels to those in Matrigel, a control group.

Further, it was confirmed that ZO-1, a marker involved in tight-junction between cells, was also well expressed in all the kidney organoids cultured in the KEM hydrogels with the respective concentrations.

It was confirmed that KI67, a marker related to organoid proliferation, was also well expressed in all the kidney organoids cultured in the KEM hydrogels with the respective concentrations.

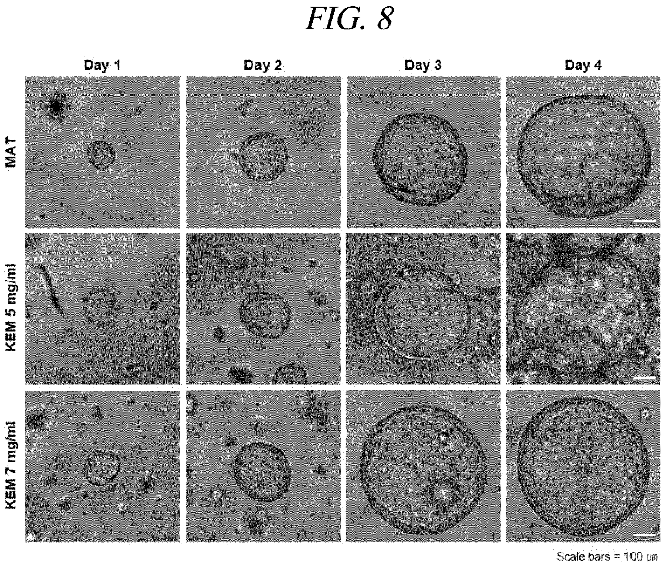

### Growth pattern of kidney organoid cultured in KEM hydrogel scaffold (FIG. 8)

From day 1 to day 4 of subculture performed on day 7 of culture, growth patterns of the kidney organoids respectively cultured in Matrigel and the KEM hydrogels (5 mg/ml and 7 mg/ml) were observed and compared.

It was confirmed that the organoids grew in the KEM hydrogels with the respective concentrations in similar patterns to that in Matrigel, and were well cultured for 4 days.

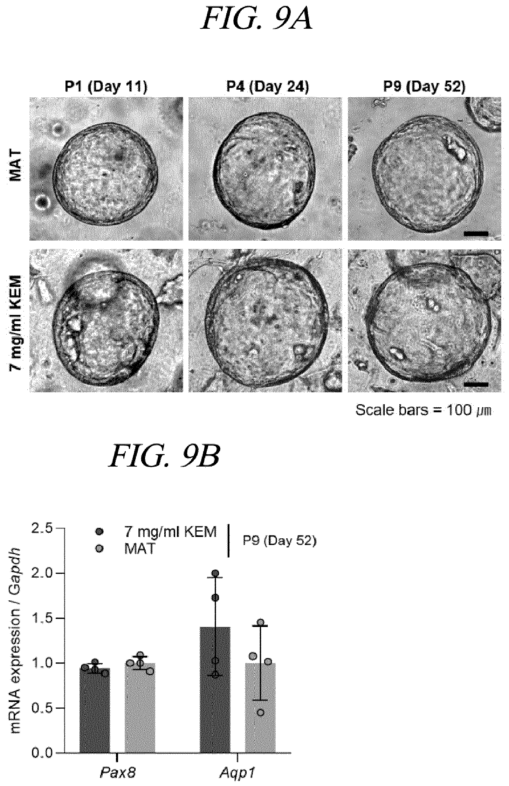

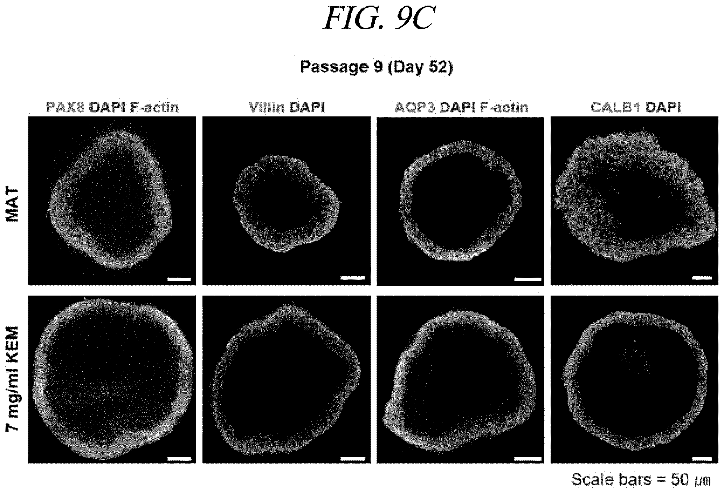

### Analysis of kidney organoid long-term cultured in KEM (FIG. 9)

(A) When kidney organoids were cultured for up to 52 days in a KEM hydrogels and Matrigel, it was confirmed that the organoids were cultured while maintaining similar spherical shapes and sizes in the both scaffolds even after 9 times of subculture.

(B) As a result of comparing the mRNA expression levels of *Pax8* (renal progenitor cell) and *Aqp1* (proximal tubule cell) of the kidney organoids which were subcultured 9 times for 52 days in the respective culture scaffolds, it was confirmed that *Pax8* was expressed at similar levels and the expression level of *Aqp1* was higher in the KEM hydrogel.

(C) Immunostaining was performed to the organoids, which were subcultured 9 times in the respective culture scaffolds, with PAX8 (renal progenitor cell), Villin (proximal tubule cell), AQP3 (collecting duct cell), and CALB1 (distal tubule cell) antibodies. As a result of the immunostaining, it was confirmed that the four types of cells constituting the renal tubule were cultured while well maintaining the expression of these markers until day 52. Also, it was confirmed that F-actin, a cytoskeleton marker involved in cell-cell interaction, was well expressed.

Accordingly, it was demonstrated that a kidney organoid can be continuously cultured for more than 2 months in a KEM hydrogel, and various cell type markers of the kidney are expressed at similar or higher levels in the KEM hydrogel than in Matrigel, a commercially available organoid culture matrix.

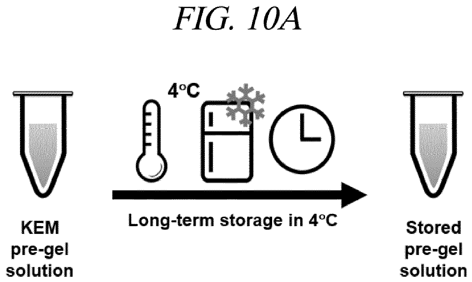

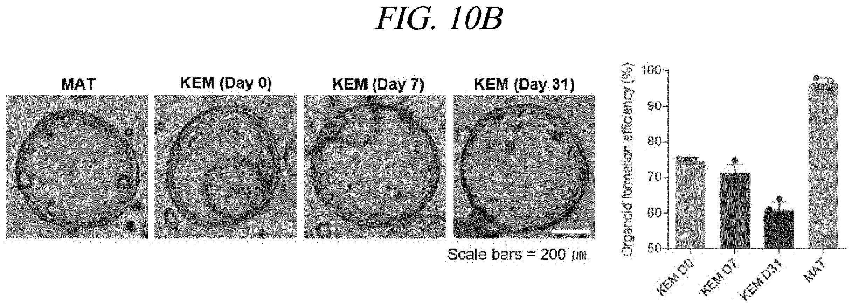

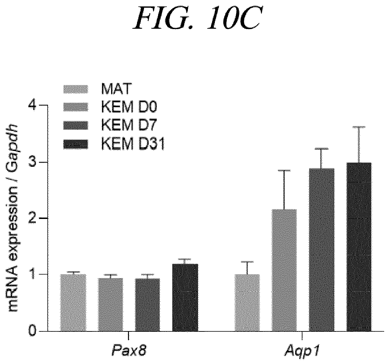

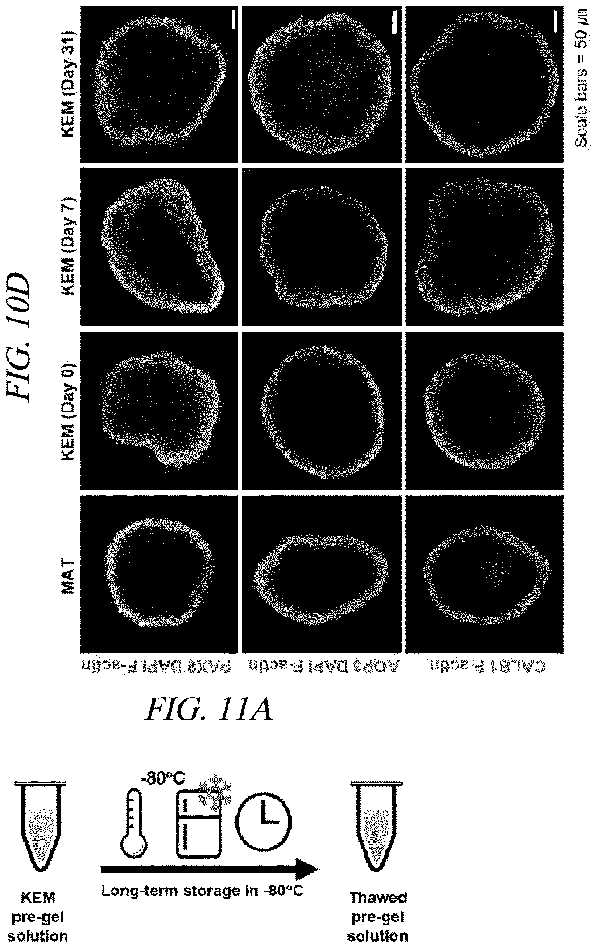

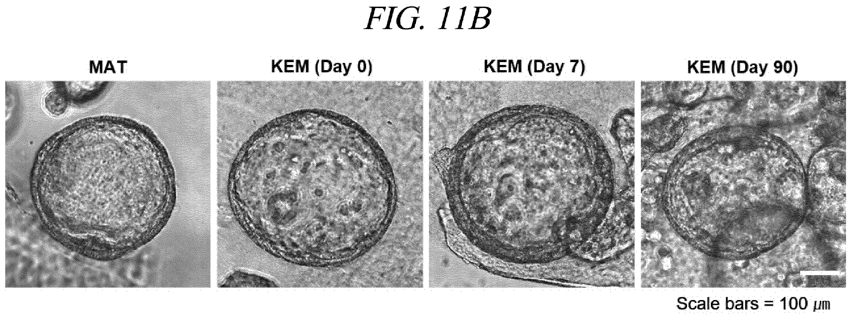

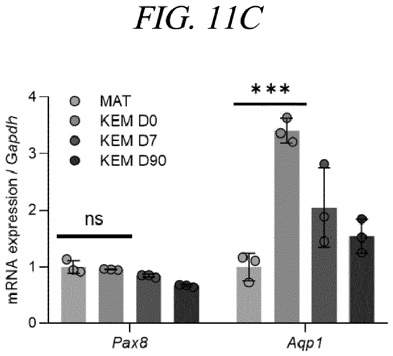

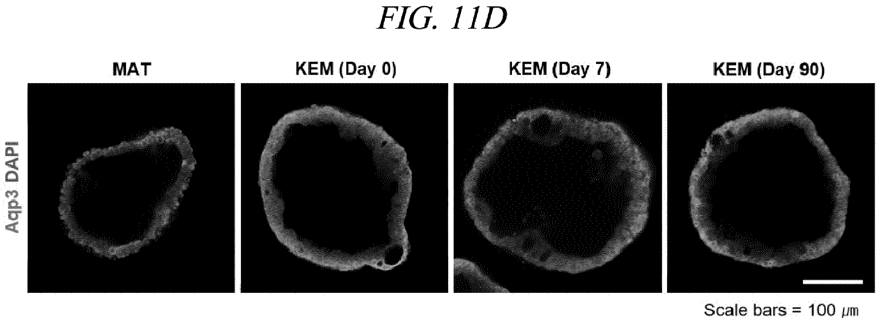

### Verification on possibility of long-term storage of KEM (FIG. 10 and FIG. 11)

First, the possibility of long-term cold storage of the KEM was verified (**FIG. 10**).
(A) A KEM solution (concentration of 7 mg/ml) was cold-stored at 4°C for up to one month and then used for kidney organoid culture to verify the possibility of long-term cold storage of a KEM hydrogel and the stability thereof.
(B) It was confirmed that kidney organoids were well formed and cultured in all of a hydrogel prepared immediately before culture with a fresh KEM solution (Day 0) and hydrogel scaffolds prepared with KEM solutions cold-stored for 1 week (Day 7) and 1 month (Day 31), respectively, at similar levels to that in Matrigel. (Images of the organoids on day 12 of culture, subcultured once) The number of organoids was measured right after subculture and after additional culture for 4 days and then expressed as a ratio.
(C) According to the result of quantitative qPCR analysis, *Pax8,* a gene related to stemness, was expressed in the KEM hydrogel group at a similar level to that in the Matrigel group. Also, the mRNA expression level of *Aqp1* (proximal tubule cell), a kidney-specific marker, was generally higher in the KEM hydrogel group than in the Matrigel group. (The analysis was performed to the organoid samples on day 12 of culture, subcultured once)
(D) As a result of immunostaining of the organoids, AQP3 (collecting duct cell), CALB1 (distal tubule cell), and PAX8 (renal progenitor cell), kidney-specific markers, were well expressed in the KEM hydrogels at similar levels to those in Matrigel.

Through this experiment, it was verified that even if the KEM hydrogel solution is cold-stored in liquid form, it can be long-term stored for at least one month without affecting its activity and function. This proves the possibility of long-term storage of the KEM hydrogel, which is important for product development.

Also, the possibility of long-term cryopreservation of the KEM was verified (**FIG. 11**).
(A) A KEM solution (concentration of 7 mg/ml) was cryopreserved at -80°C for up to 3 months and then thawed and used for kidney organoid culture to verify the possibility of long-term storage of a KEM and the stability thereof.
(B) It was confirmed that kidney organoids were well formed and cultured in all of a hydrogel prepared immediately before culture with a fresh KEM solution (Day 0) and hydrogel culture scaffolds prepared with KEM solutions cryopreserved for 1 week (Day 7) and 3 months (Day 90), respectively, at similar levels to that in Matrigel. (Images of the organoids on day 12 of culture, subcultured once)
(C) According to the result of quantitative qPCR analysis, *Pax8,* a gene related to stemness, was expressed in the KEM hydrogel group at a similar level to that in the Matrigel group. Also, the mRNA expression level of *Aqp1* (proximal tubule cell), a kidney-specific marker, was generally higher in the KEM hydrogel group than in the Matrigel group. (The analysis was performed to the organoid samples on day 12 of culture, subcultured once)
(D) As a result of the immunostaining, AQP3 (collecting duct cell), a kidney-specific marker, was well expressed in the KEM hydrogels at a similar level to that in Matrigel.

**Through** this experiment, it was verified that even if the KEM hydrogel solution is frozen and stored under cryopreservation conditions (-80°C), it can be long-term stored stably for at least 3 months without affecting its activity and function.

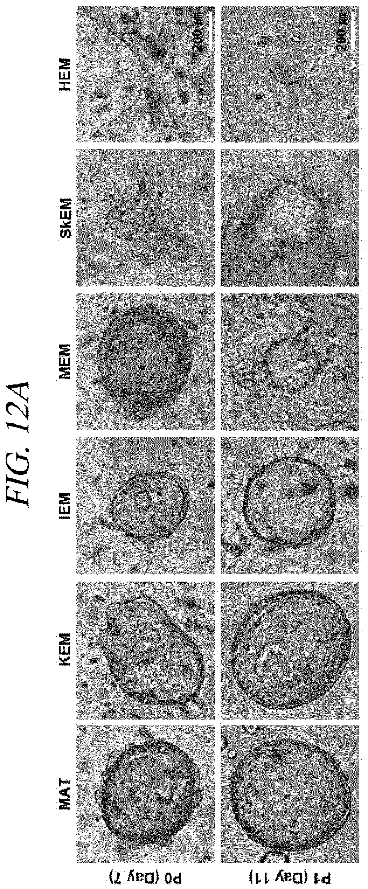

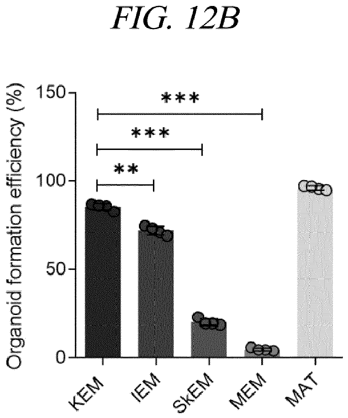

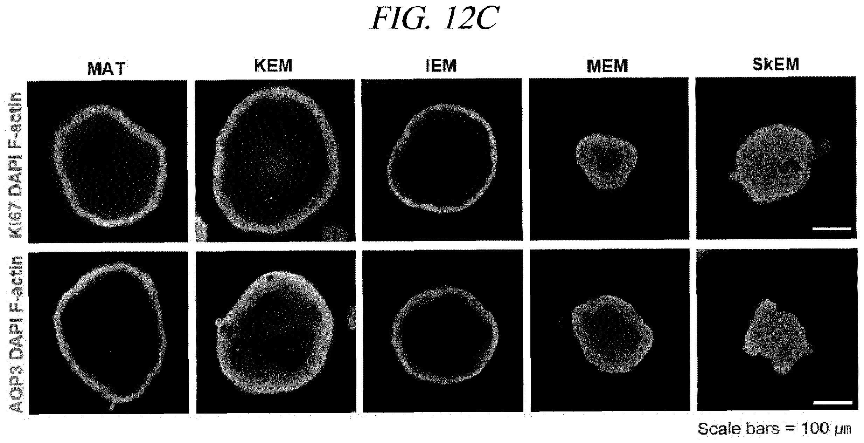

### Verification on tissue-specific effect of KEM for kidney organoid culture (FIG. 12)

Kidney organoids were cultured in decellularized scaffolds derived from other organs and then compared and analyzed to verify the tissue-specific effect of kidney-specific extracellular matrix components contained in the KEM scaffold on the formation and development of a kidney organoid.
(A) As a result of culturing kidney organoids in decellularized hydrogel scaffolds derived from kidney (KEM), intestine (IEM), muscle (MEM), skin (SkEM), and heart (HEM), respectively, it was confirmed that kidney organoids were not properly formed in the skin- and heart-derived decellularized scaffolds on day 7. Further, when subculture was performed once and additional culture was performed for 4 days (for a total of 11 days of culture), it was observed that organoids were formed in small sizes in all of the decellularized tissue scaffolds except the decellularized kidney tissue-derived scaffold, and an organoid was not formed in the heart-derived scaffold.
(B) As a result of quantitative analysis of kidney organoid formation efficiency, it was confirmed that the formation efficiency was the highest in the KEM scaffold, and the formation efficiency was significantly reduced in the decellularized scaffolds derived from the other organs. The number of organoids was measured right after subculture and after additional culture for 4 days and then expressed as a ratio to measure the formation efficiency.
(C) Immunostaining was performed using KI67, an antibody related to cell proliferation, and AQP3, an antibody related to collecting duct cells, to compare the marker protein expression levels of the kidney organoids cultured for 11 days in the decellularized hydrogel scaffolds derived from the respective organs. It was confirmed that the kidney organoid cultured in the KEM hydrogel scaffold exhibited the most distinct marker expression, but only relatively minute expression was observed or the structure was not properly formed in the other organoids cultured in the other tissue-derived scaffolds.

Through this experiment, the tissue-specific effect of the KEM hydrogel on the formation and development of a kidney organoid was verified.

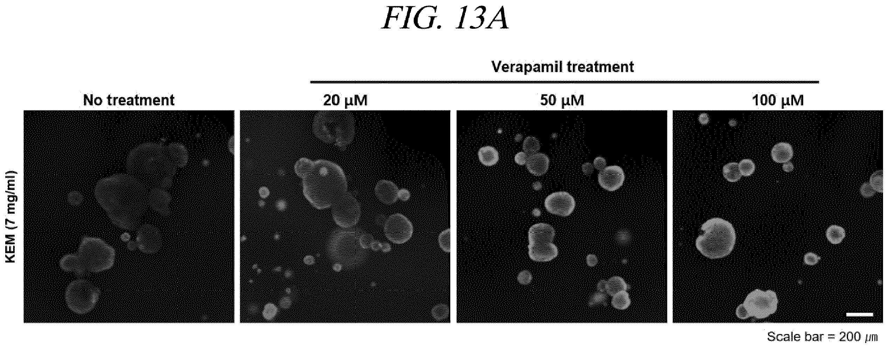

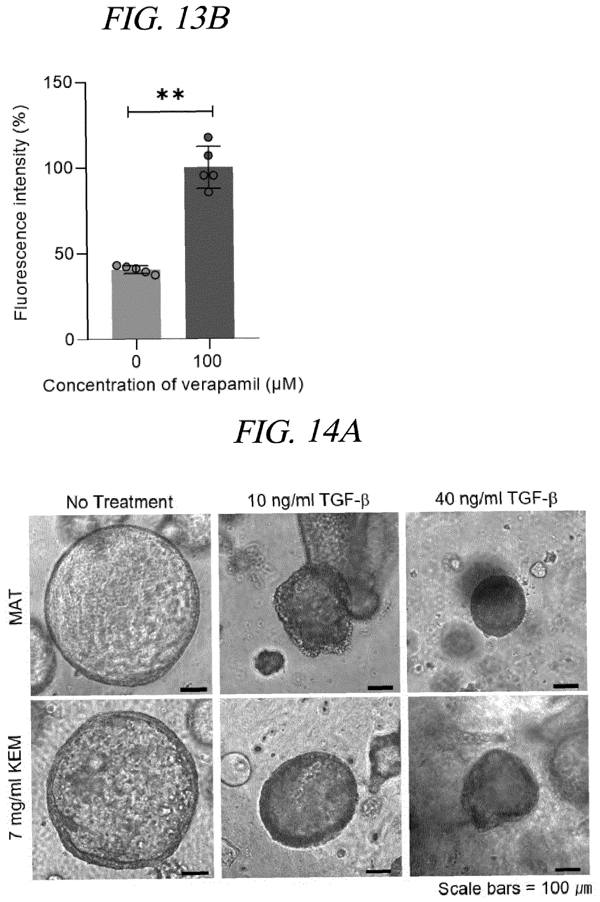

### Analysis of functionality of kidney organoid cultured in KEM (FIG. 13)

An analysis was performed to determine whether a kidney organoid cultured in the KEM hydrogel can well implement kidney function in vivo. In order to check the functionality of the P-glycoprotein (P-gp) xenobiotics efflux pump present in the proximal tubular cells, one of several cell types constituting the kidney organoid, after the kidney organoid was treated with verapamil, a P-gp inhibitor, and exposed to calcein AM, the degree of accumulation of calcein AM in the cells was measured with fluorescence.

(A) It was confirmed that in the kidney organoid not treated with verapamil, P-gp secreted calcein out of the cells and fluorescence signals were hardly accumulated in the organoid (No treatment group). It was confirmed that as the treatment concentration of the verapamil increased, calcein was accumulated in the cells and fluorescence was expressed in the organoid at a relatively high level.

(B) As a result of quantification of calcein fluorescence intensity in the kidney organoid not treated with verapamil and in the kidney organoid treated with 100 µM verapamil, it was confirmed that the fluorescence intensity was significantly increased in the kidney organoid treated with verapamil.

Accordingly, it can be seen that the kidney organoid cultured in the KEM hydrogel functions as an efflux pump to discharge foreign matters.

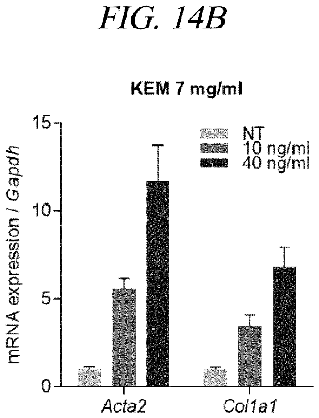

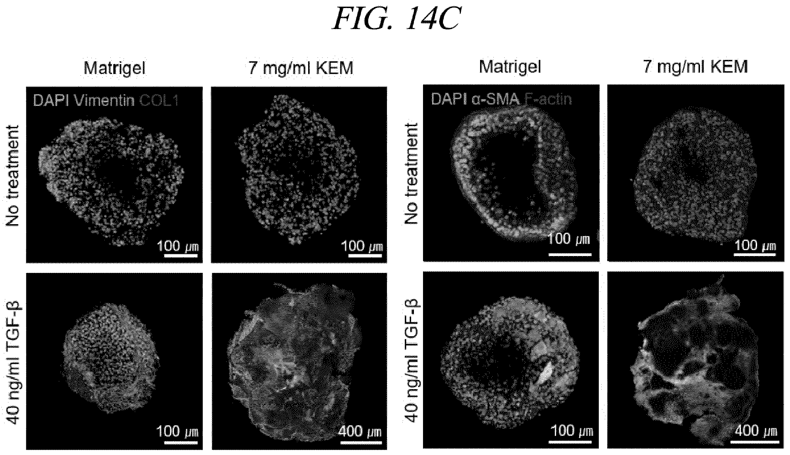

### Construction of kidney fibrosis model using kidney organoid cultured in KEM (FIG. 14)

A kidney fibrosis model was constructed using a kidney organoid cultured in the KEM hydrogel. To this end, kidney fibrosis was induced by treatment of a mouse kidney organoid with TGF-b. On day 9 of kidney organoid culture (subculture once on day 7 and additional culture for 2 days), the kidney organoids were treated with each concentration of TGF-β for 3 days and then analyzed on day 12. A kidney organoid not treated with TGF-β (NT, No treatment) was compared as a control group.
(A) As a result of the treatment with each concentration of TGF-β to construct a kidney fibrosis model, it was confirmed that the kidney organoid not treated with TGF-β grew well, but the kidney organoids treated with TGF-β exhibited morphological changes and did not grow well.
(B) As a result of qPCR analysis of *Acta2* and *Col1a1,* kidney fibrosis-related markers, it was confirmed that the mRNA expression levels of the fibrosis markers increased in proportion to the increase in treatment concentration of TGF-β.
(C) Immunostaining was performed to confirm the expression of Vimentin, COL1 (collagen type 1), and α-SMA (alpha smooth muscle actin), kidney fibrosis-related markers. When a kidney organoid cultured in a 7 mg/ml KEM hydrogel was treated with TGF-β, it was confirmed that the KEM hydrogel contracted and fibrosis-induced organoids agglomerated in a mass. As a result of immunostaining, it was confirmed that the expression of all fibrosis markers was increased in the kidney organoids treated with TGF-β and all of the kidney organoids in Matrigel and the KEM scaffolds exhibited similar fibrosis marker expression patterns.

Through this experiment, it is possible to confirm the possibility of construction of a kidney fibrosis model based on the kidney organoid cultured in the KEM hydrogel scaffold.

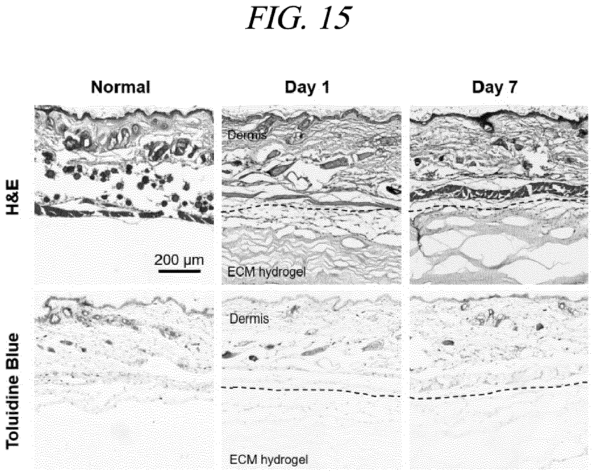

### Verification on biocompatibility of KEM (FIG. 15)

In order to verify whether a KEM hydrogel scaffold is suitable as a material for kidney organoid transplantation, a 5 mg/ml KEM scaffold was transplanted into the mouse subcutaneous tissue, followed by histological analysis. H&E staining was performed to check the degree of infiltration of inflammatory cells into the transplanted KEM hydrogel scaffold, and toluidine blue staining was performed to check the presence or absence of mast cells caused by an immune response. As a result of the analysis, no inflammatory cells were found at the site where the KEM hydrogel was transplanted, which verifies that immune or inflammatory response in vivo is hardly induced when a KEM hydrogel is transplanted.

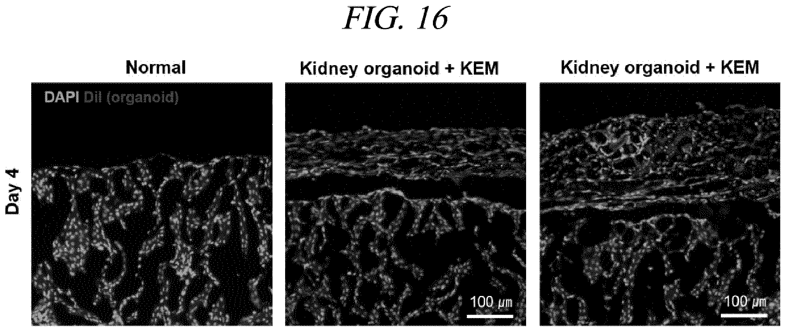

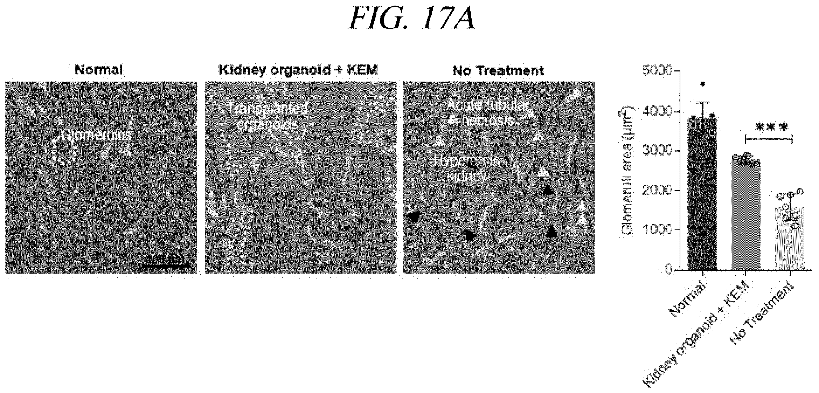

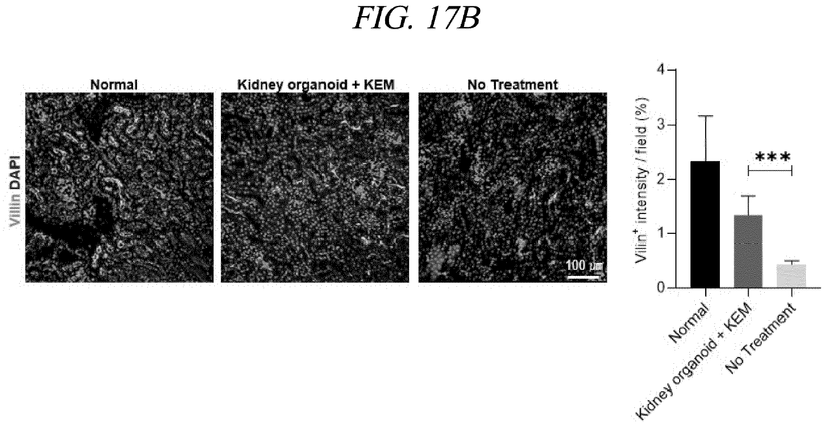

### Result of in vivo transplantation of kidney organoid using KEM (FIG. 16 and FIG. 17)

In order to verify whether a KEM hydrogel can be used as a material for organoid transplantation, a kidney organoid was transplanted into the mouse kidney capsule using a KEM hydrogel. To check the engraftment and presence of the transplanted cells, the kidney organoid labeled with Dil fluorescent dye was used. To adjust the viscosity of the hydrogel for improvement in efficiency of transplantation into the kidney tissue, the KEM hydrogel was mixed at a ratio of 1:20 (v/v) (KEM: culture medium composition) and transplanted into a living body.

To observe the degree of engraftment of the transplanted kidney organoid in vivo, the kidney tissue was obtained on day 4 of transplantation, followed by histological analysis. As a result, as shown in **FIG. 16**, the presence of a fluorescence signal inside the kidney capsule was confirmed. Therefore, it was confirmed that the transplanted organoid was well engrafted in the tissue.

Animal tests and histological analysis were performed to verify whether a KEM hydrogel scaffold can be used as a material for kidney organoid transplantation. An acute kidney injury (AKI) model was induced by intraperitoneal injection of 10 mg/kg cisplatin once, and on the next day, 600 to 700 kidney organoids were transplanted into the mouse kidney capsule using 25 µl of a 7 mg/ml KEM hydrogel to improve the transplantation efficiency and engraftment. On day 14 of transplantation, the kidney tissues were collected from each group of mice and subjected to H&E staining and immunostaining analysis.

Then, the possibility of transplantation of a kidney organoid in vivo in a kidney injury animal model was confirmed (**FIG. 17**).
(A) As a result of H&E staining, acute tubular necrosis (ATN) and hyperemic kidney, which are typical symptoms of acute kidney injury, were widespread in the damaged kidney tissue into which an organoid was not transplanted. However, when the kidney organoids were transplanted using the KEM scaffold, it was confirmed that the kidney organoids were well engrafted in the cortical region of the damaged kidney tissue and the damaged area was significantly reduced compared with a control kidney tissue that was not treated. Also, as a result of quantitative analysis of the glomerular area to check the degree of contraction of the glomerular structure, which is one of the symptoms of acute kidney injury, it was confirmed that the kidney tissue transplanted with the kidney organoids maintained a less damaged glomerular structure than the kidney tissue into which an organoid was not transplanted.
(B) Immunostaining for Villin, a proximal tubule marker, was performed to check the degree of apoptosis of the proximal tubule, which is one of the main symptoms of acute renal injury, in each condition. As a result, it was confirmed that the proximal tubule accounted for a significantly higher portion in the kidney tissue transplanted with the organoids than in the damaged kidney tissue into which an organoid was not transplanted. Therefore, it was confirmed that the transplanted organoids were engrafted in the damaged site and the proximal tubule was regenerated.

Through this experiment, it was verified that the KEM hydrogel scaffold can be used as a material for organoid transplantation to treat a kidney disease model and can improve the engraftment efficiency of an organoid in damaged tissue and induce a recovery of kidney structure.

The above description of the present disclosure is provided for the purpose of illustration, and it would be understood by a person with ordinary skill in the art that various changes and modifications may be made without changing technical conception and essential features of the present disclosure. Thus, it is clear that the above-described examples are illustrative in all aspects and do not limit the present disclosure.

## Claims

1. A scaffold for culture and transplantation of a kidney organoid using a kidney extracellular matrix (KEM).

2. The scaffold of Claim 1,
wherein the KEM is prepared by using a mixed solution of Triton X-100 and ammonium hydroxide.

3. The scaffold of Claim 1,
wherein a concentration of the KEM in the scaffold is from 1 mg/ml to 10 mg/ml.

4. A method of preparing a scaffold for culture and transplantation of a kidney organoid, comprising:
a process (1) of crushing an isolated kidney tissue; and
a process (2) of treating the crushed kidney tissue with Triton X-100 and ammonium hydroxide for decellularization to prepare a decellularized KEM.

5. The method of preparing a scaffold for culture and transplantation of a kidney organoid of Claim 4, further comprising:
after the process (2), a process (3) of lyophilizing the decellularized KEM to prepare a lyophilized KEM.

6. The method of preparing a scaffold for culture and transplantation of a kidney organoid of Claim 5, further comprising:
after the process (3), a process (4) of forming a scaffold for culture and transplantation of a kidney organoid in the form of a hydrogel with the lyophilized KEM.

7. The method of preparing a scaffold for culture and transplantation of a kidney organoid of Claim 6,
wherein in the process (4), the lyophilized KEM is dissolved in a pepsin solution and a pH of the solution is adjusted to form the hydrogel.

8. A method of culturing a kidney organoid in the scaffold of Claim 1 or in a scaffold prepared by the preparation method of Claim 4.
